# EUROPÄISCHE PATENTANMELDUNG

(11) **EP 0 125 506 A1**
(43) Veröffentlichungstag der Anmeldung: **21.11.1984**
(21) Anmeldenummer: 84104189.0
(22) Anmeldetag: 17.11.1981
(51) Int. Cl.: C07C 79/35, C07C 76/00

(54) **5-(2,6-Dichlor-4-trifluormethyl-phenoxy)-2-nitrobenzaldehyd-diacetacylal**

(30) Priorität: 28.11.1980 DE 3044810
(62) Teilanmeldung aus: 81109729.4
(71) Anmelder: BAYER AG, 51368 Leverkusen (DE)
(72) Erfinder: Förster, Heinz, Dr., D-5600 Wuppertal 1 (DE); Eue, Ludwig, Dr., D-5090 Leverkusen 1 (DE); Schmidt, Robert R., Dr., D-5060 Bergisch Glasbach 2 (DE)

(57) **Zusammenfassung**

5-(2,6-Dichlor-4-trifluormethyl-phenoxy)-2-nitro-benzaldehyd-diacetacylal der Formel 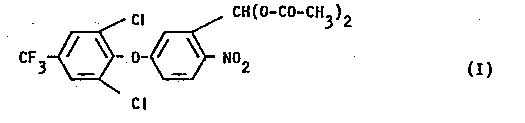 ein Verfahren zu dessen Herstellung und dessen Verwendung als Zwischenprodukt zur Synthese von Phenoxyzimtsäure-Derivaten mit herbiziden Eigenschaften.

## Beschreibung

Die vorliegende Erfindung betrifft das neue 5-(2,6-Dichlor-4-trifluormethyl-phenoxy)-2-nitro-benzaldehyd-diacetacylal, ein Verfahren zu dessen Herstellung und dessen Verwendung als Zwischenprodukt zur Synthese von substituierten Phenoxyzimtsäure-Derivaten mit herbiziden Eigenschaften.

Es ist bereits bekannt geworden, daß 5-(2,6-Dichlor-4-trifluormethylphenoxy)-2-nitro-benzaldehyd als Zwischenprodukt zur Herstellung von bestimmten Diphenylether-Derivaten mit herbizider Wirksamkeit eingesetzt werden kann (vgl. DE-A 30 17 795). Eine spezielle Synthese des betreffenden Aldehydes ist jedoch bisher noch nicht beschrieben worden.

Es wurde nun das neue 5-(2,6-Dichlor-4-trifluormethyl- phenoxy)-2-nitro-benzaldehyd-diacetacylal der Formel 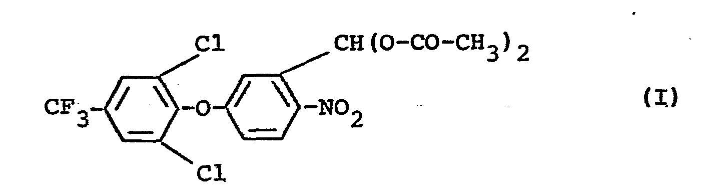 gefunden.

Weiterhin wurde gefunden, daß man das neue 5-(2,6-Dichlor-4-trifluormethyl-phenoxy)-2-nitro-benzaldehyd-diacetacylal erhält, wenn man 5-(2,6-Dichlor-4-trifluormethyl-phenoxy)-benzaldehyd der Formel 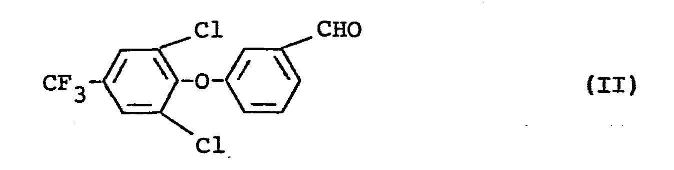 mit Acetanhydrid gegebenenfalls in Gegenwart eines Verdünnungsmittels umsetzt und anschließend mit Salpetersäure nitriert.

Schließlich wurde gefunden, daß sich 5-(2,6-Dichlor-4-trifluormethyl-phenoxy)-2-nitro-benzaldehyd-diacetacylal der Formel (I) sehr gut-als Zwischenprodukt zur Herstellung von Phenoxyzimtsäure-Derivaten mit herbiziden Eigenschaften verwenden läßt.

Überraschenderweise sind ausgehend von der erfindungsgemäßen Verbindung der Formel (I) über den 5-(2,6-Dichlor-4-trifluormethyl-phenoxy)-2-nitro-benzaldehyd neue Phenoxyzimtsäure-Derivate zugänglich, die eine bessere herbizide Wirksamkeit besitzen als konstitutionell ähnliche, vorbekannte Verbindungen gleicher Wirkungsrichtung.

Dir bei dem erfindungsgemäßen Verfahren als Ausgangsstoff benötigte 3-(2,6-Dichlor-4-trifluormethyl- phenoxy)-benzaldehyd der Formel (II) wurde bisher noch nicht in der Literatur beschrieben. Man erhält diese Verbindung, wenn man 3-_{H}ydroxy-benzaldehyd mit 3,4,5-Trichlor-benzotrifluorid in Gegenwart eines Säurebindemittels, wie z.B. Kaliummethylat und in Gegenwart von Verdünnungsmitteln, wie z.B. Dimethylsulfoxid und Toluol, bei Temperaturen zwischen 50 und 120°C umsetzt. Die Aufarbeitung kann nach üblichen Methoden durchgeführt werden, beispielsweise durch Abdestillieren der flüchtigen Komponenten, Digerieren des Rückstandes mit Toluol, Filtration, Waschen und Einengen des Filtrats. Der hierbei erhaltene Rückstand enthält im wesentlichen das Produkt der Formel (II), welches über sein Bisulfit-Addukt gereinigt werden kann.

Als Verdünnungsmittel kann bei der Durchführung des erfindungsgemäßen Verfahrens z.B. Methylenchlorid eingesetzt werden.

Die erfindungsgemäße Umsetzung der Verbindung der Formel (II) mit Acetanhydrid erfolgt bei Temperaturen zwischen 10 und 80°C. Die anschließende Umsetzung mit Salpetersäure wird bei Temperaturen zwischen -10°C und +20°C vorgenommen.

Nach Beendigung der Reaktion wird mit Wasser verdünnt, gegebenenfalls das nicht-wäßrige Verdünnungsmittel abgetrennt oder abdestilliert und das kristallin anfallende Produkt der Formel (I) durch Filtration isoliert.

Das 5-(2,6-Dichlor-4-trifluormethyl-phenoxy)-2-nitrobenzaldehyd-diacetacylal der Formel (I) läßt sich durch Verseifung in den entsprechenden Aldehyd der Formel 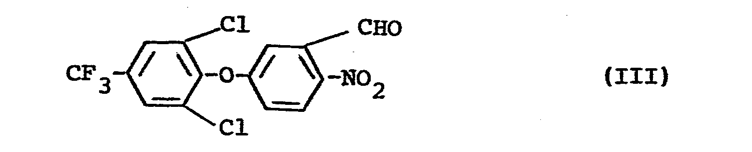 überführen, der seinerseits durch Umsetzung mit MethylenVerbindungen in Phenoxyzimtsäure-Derivate der Formel 1 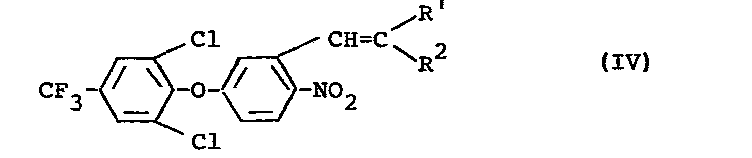 in welcher
R¹ für Wasserstoff, Cyano, C₁-C₄-Alkyl, Acetyl oder C₁-C₄-Alkoxycarbonyl steht und
_{R}² für Cyano, C1-C4-Alkoxy-carbonyl oder COOM steht, wobei
M für Wasserstoff, Natrium, Kalium, ein Magnesium- oder Calciumionenäquivalent steht,

umwandeln.

Der Aldehyd der Formel (III) läßt sich herstellen, indem man das 5-(2,6-Dichlor-4-trifluormethyl-phenoxy)-2-nitro benzaldehyd-diacetacylal der Formel 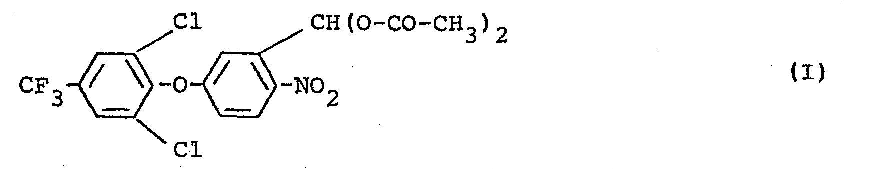 mit wäßrigen Alkalilaugen, wie z.B. Natronlauge, gegebenenfalls in Gegenwart eines weiteren Verdünnungsmittels, wie z.B. Methanol, bei Temperaturen zwischen 10 und 80°C umsetzt, danach mit Wasser verdünnt und das kristalline Produkt der Formel (III) durch Filtration isoliert.

Die Phenoxyzimtsäure-Derivate der Formel (IV) lassen sich herstellen, indem man den 5-(2,6-Dichlor-4-trifluormethyl-phenoxy)-2-nitro-benzaldehyd der Formel 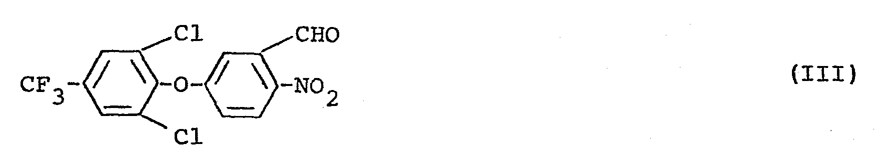 mit Methylenverbindungen der Formel 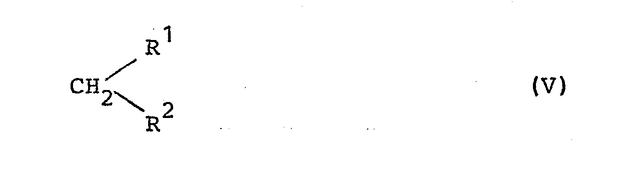 in welcher
_{R}¹ und _{R}² die oben angegebene Bedeutung haben,

gegebenenfalls in Anwesenheit eines Katalysators und gegebenenfalls in Anwesenheit eines Verdünnungsmittels umsetzt und gegebenenfalls die hierbei gebildeten Ver--bindungen der Formel (IV) nach üblichen Methoden zur Abwandlung der Reste R 1 und/oder R² in andere Verbindungen der Formel (IV) umwandelt.

Als Beispiele für Verbindungen der Formel (V), die bei dem Verfahren zur Herstellung der Phenoxyzimtsäure-Derivate der Formel (IV) als Ausgangsstoffe benötigt werden, seien genannt:
Malonsäure, deren Natrium-, Kalium-, Calcium- und Magnesiumsalz sowie deren Methylester, Ethylester, n- und iso-Propylester, n-, iso-, sek- und tert.-Butylester, Malonsäuredinitril, Cyanessigsäure, deren Natrium-, Kalium-, Calcium- und Magnesiumsalz sowie deren Methylester, Ethylester, n- und iso-Propylester, n-, iso-, sek.- und tert.-Butylester, ferner Acetessigsäuremethylester, -ethylester, -n- und -iso-propylester, -n-, -iso-, sek.- und -tert.-butylester.

Die Verbindungen der Formel (V) sind bekannt.

Als Möglichkeiten zur Umwandlung der nach dem obigen Verfahren gebildeten Phenoxyzimtsäure-Derivate der Formel (IV) seien genannt:
1. die Decarboxylierung, welche bei Verwendung von Malonsäure oder von Cyanessigsäure als Ausgangsstoffe im allgemeinen in situ abläuft, ohne daß die entsprechenden Primärprodukte isoliert werden;
2. die Salzbildung, beispielsweise durch Umsetzung der entsprechenden Säuren mit basischen Alkali-oder Erdalkaliverbindungen;
3. die Veresterung, beispielsweise durch Umsetzung der entsprechenden Säuren mit Alkoholen in Gegenwart von Basen, wie z.B. Diazabicycloundecen, oder durch Umsetzung mit geeigneten Alkylhalogeniden.

Das Verfahren zur Herstellung der neuen Phenoxyzimtsäurederivate der Formel (IV) wird in Gegenwart eines Katalysators durchgeführt. Als solche dienen Verbindungen, welche für das Beschleunigen von Kondensationsreaktionen zwischen Aldehyden und aktivierten Methylenverbindungen (Aldolkondensationen) geeignet sind. Vorzugsweise werden aliphatische, aromatische oder heterocyclische Amine, wie z.B. Trimethylamin, Triethylamin, Pyrrolidin, Piperidin, Dimethylanilin, Dimethylbenzylamin, Diazabicyclooctan, Diazabicyclononen, Diazabicycloundecen und Pyridin, gegebenenfalls als Salze von schwachen Säuren, wie z.B. von Essigsäure verwendet. Zu den Katalysatoren gehören auch Aminocarbonsäuren, wie z.B. ß-Alanin. Einzelne dieser Katalysatoren können, wie z.B. Pyridin, auch zweckmäßig als Verdünnungsmittel verwendet werden.

Als Verdünnungsmittel kommen bei der Herstellung der Phenoxyzimtsäure-Derivate der Formel (IV) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methyl-isopropyl- und Methyl-isobutylketon, Ester, wie Essigsäure-methylester und.-ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperatur kann bLi dem Verfahren zur Herstellung der Phenoxyzimtsäure-Derivate der Formel (IV) innerhalb eines größeren Bereichs variiert werden. Im allgemeinen arbeitet man zwischen 0 und 200°C, vorzugsweise zwischen 20 und 150°C. Das Verfahren wird im allgemeinen bei Normaldruck durchgeführt.

Pro Mol Phenoxybenzaldehyd der Formel (III) werden bei der Durchführung des Verfahrens zur Herstellung der Phenoxyzimtsäure-Derivate der Formel (IV) zwischen 0,9 und 1,5 Mol, vorzugsweise zwischen 1,0 und 1,2 Mol Methylenchlorid der Formel (V) eingesetzt. Zur Durchführung dieses Verfahrens werden im allgemeinen die Reaktionskomponenten der Formeln (III) und (V) sowie der Katalysator und das Verdünnungsmittel bei Raumtemperatur zusammengegeben und das Gemisch wird dann bei erhöhter Temperatur, vorzugsweise zwischen 50 und 120°C bis zum Ende der Umsetzung gerührt. Das bei der Reaktion gebildete Wasser wird gegebenenfalls über einen Wasserabscheider entfernt.

Die Aufarbeitung kann nach üblichen Methoden durchgeführt werden. Soweit die Produkte bei Raumtemperatur kristallin sind, erhält man sie durch Verdünnen der Reaktionsmischung mit Wasser, gegebenenfalls Ansäuern und Filtrieren.

Es kann jedoch auch, wie üblich, durch Verdünnen mit Wasser, Schütteln mit einem mit Wasser praktisch nicht mischbaren organischen Lösungsmittel, wie z.B. Toluol, Abtrennen und Waschen der organischen Phase mit Wasser sowie Abdestillieren des organischen Lösungsmittels aufgearbeitet werden.

Die Phenoxyzimtsäure-Derivate der Formel (IV) zeichnen sich durch hervorragende herbizide Wirksamkeit aus und zeigen eine gute Selektivität in verschiedenen Kulturen, wie Sojabohnen, Mais und Getreide. Neben der hohen Wirkung gegen breitblättrige Unkräuter besitzen die Verbindungen der Formel (IV) auch eine gute Wirkung gegen'Gräser, vor allem gegen Hirsearten; sie zeigen bei Nachauflaufanwendung pflanzenwuchsregulierende, insbesondere wuchshemmende Eigenschaften und können beispielsweise als Baumwolldefoliants genutzt werden.

Die Herstellung des erfindungsgemäßen 5-(2,6-Dichlor-4-trifluormethyl-phenoxy)-2-nitro-benzaldehyd-diacetacylals der Formel (I) und dessen Einsatz als Zwischenprodukt zur Synthese des 5-(2,6-Dichlor-4-trifluormethyl- phenoxy)-2-nitro-benzaldehyds geht aus den folgenden Beispielen hervor. Ferner zeigen diese Beispiele auch die Verwendung des Aldehyds der Formel (III) als Zwischenprodukt zur Herstellung von Phenoxyzimtsäure-Derivaten der Formel (IV) und deren Verwendung als Herbizide.

### Herstellungsbeispiele

### Beispiel 1

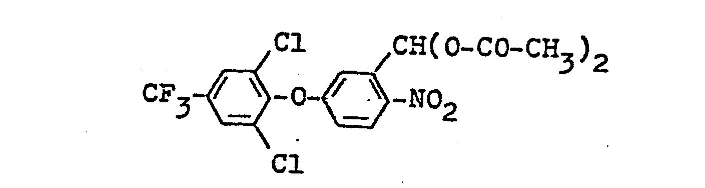

34 g 3-(2,6-Dichlor-4-trifluormethyl-phenoxy)-benzaldehyd und 42,8 g Acetanhydrid werden in 100 ml Methylenchlorid gelöst, und die Lösung wird 1 Stunde bei 40°C gerührt. Nach dem Abkühlen auf 20°C werden 2 g Schwefelsäure dazu gegeben. Dann werden bei 2 bis 5°C 10 g 70 %ige Salpetersäure zugetropft, und die Reaktionsmischung wird 4 Stunden bei 0 bis 5°C gerührt. Zur Aufarbeitung wird mit 1 1 Wasser verdünnt, das Methylenchlorid abdestilliert und vom kristallinen Produkt abgesaugt. Man erhält 80 g 5-(2,6-Dichlor-4-trifluormethyl-phenoxy)-2-nitro-benzaldehyd-diacetacylal vom Schmelzpunkt 132°C.

### Herstellung des Ausgangsproduktes der Formel

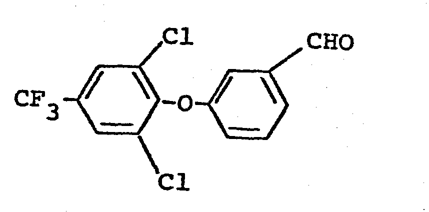

Eine Mischung aus 123 g Kaliumhydroxid, 122 g 3-Hydroxybenzaldehyd und 1 1 Methanol wird bis zur Bildung einer Lösung gerührt. Dann wird das Methanol im Vakuum sorgfältig abdestilliert. Der Rückstand wird in 1,8 1 Dimethylsulfoxid gelöst. Dazu werden 300 ml Toluol gegeben und langsam wieder abdestilliert. Dabei werden 536 g 3,4,5-Trichlor-benzotrifluorid zugetropft. Das Reaktionsgemisch wird noch 9 Stunden bei 90 bis 95°C gerührt. Anschließend wird das Lösungsmittel im Vakuum abdestilliert, der Rückstand mit 1,2 1 Toluol verrührt und abgesaugt. Das Filtrat wird alkalisch und neutral gewaschen und eingeengt. Man erhält 430 g (64 % der Theorie) 3-(2,6-Dichlor-4-trifluor-methyl- phenoxy)-benzaldehyd vom Schmelzpunkt 54°C.

### Beispiel 2

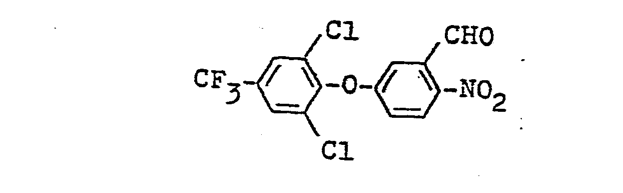

Eine Mischung aus 12,1 g 5-(2,6-Dichlor-4-trifluormethyl- phenoxy)-2-nitro-benzaldehyd-diacetacylal, 40 ml Methanol, 30 ml Wasser und 7 ml 4 %iger wäßriger Natronlauge wird 90 Minuten bei 40°C gerührt, dann in Wasser gegossen und abgesaugt. Man erhält 9,0 g (95 % der Theorie) 5-(2,6-Dichlor-4-trifluormethyl-phenoxy)-2-nitro-benzaldehyd vom Schmelzpunkt 115°C.

### Beispiel 3

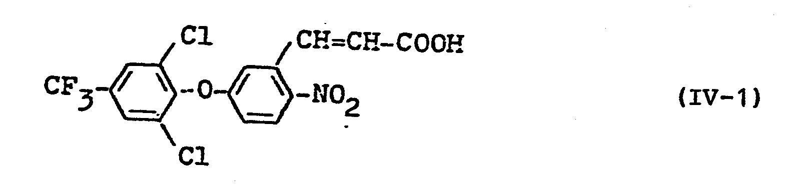

270 g 5-(2,6-Dichlor-4-trifluormethyl-phenoxy)-2-nitrobenzaldehyd und 6 g Piperidin werden bei 25°C zu 89,5 g Malonsäure in 570 ml Pyridin gegeben. Die Reaktionsmischung wird 5 Stunden bei 85 bis 90°C gerührt und weitere 90 Minuten unter Rückfluß erhitzt, dann auf Wasser gegossen, mit Salzsäure angesäuert und abgesaugt. Das kristalline Produkt wird aus n-Butanol umkristallisiert. Man erhält 200 g (66 % der Theorie) 3-(5-(2,6-Dichlor-4-trifluormethyl-phenoxy)-2-nitrophenyl)-propensäure vom Schmelzpunkt 192°C in Form gelber Kristalle.

Nach der im Beispiel 3 angegebenen Methode wird auch der in dem folgenden Beispiel aufgeführte Stoff der Formel (IV) hergestellt:

### Beispiel 4

Unter Verwendung von Cyanessigsäure: 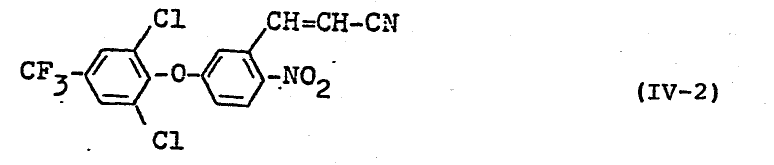 Schmelzpunkt: 142°C

### Beispiel 5

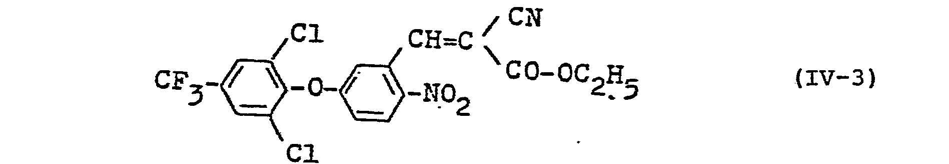

19 g 5-(2,6-Dichlor-4-trifluormethyl-phenoxy)-2-nitrobenzaldehyd, 5,7 g Cyanessigsäuremethylester, 0,6 g Essigsäure, 0,3 g Piperidin und 120 ml Toluol werden vermischt und 6 Stunden am Wasserabscheider zum Sieden erhitzt. Dann werden 200 ml Toluol dazu gegeben und die organische Phase wird mit Wasser gewaschen. Von der organischen Phase wird das Lösungsmittel abdestilliert und der Rückstand durch Digerieren mit Methanol zur Kristallisation gebracht. Man erhält 12,5 g (53 % der Theorie) 2-Cyano-3-(5-(2,6-dichlor-4-trifluormethylphenoxy)-2-nitro-phenyl)-propensäure- ethylester vom Schmelzpunkt 99°C in Form blaßgelber Kristalle.

Nach der im Beispiel 5 angegebenen Methode werden auch die in den folgenden Beispielen aufgeführten Stoffe der Formel (IV) hergestellt:

### Beispiel 6

Unter Verwendung von Malodinitril: 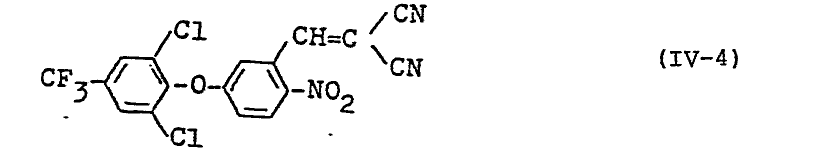 Schmelzpunkt: 114°C.

### Beispiel 7

Unter Verwendung von Cyanessigsäuremethylester: 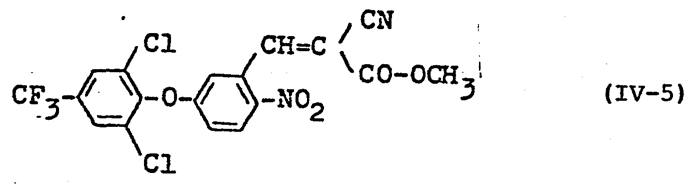 Schmelzpunkt: 148°C.

### Beispiel 8

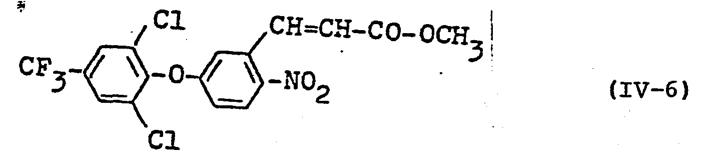

42,2 g 3-(5-(2,6-Dichlor-4-trifluormethyl-phenoxy)-2-nitro-phenyl)-propensäure (Verbindung IV-1) werden in 120 ml Aceton vorgelegt und bei 20°C mit 18,7 g Diaza₋ bicycloundecen (DBU) versetzt. Nach 30 Minuten werden 35 g Methyliodid tropfenweise dazugegeben.

Das Reaktionsgemisch wird 15 Stunden bei 50 bis 60°C gerührt mit Wasser und Methylenchlorid verdünnt; die organische Phase wird mit verdünnter Natronlauge und dann mit verdünnter Salzsäure ausgeschüttelt, mit Wasser gewaschen, getrocknet und filtriert. Vom Filtrat wird das Lösungsmittel abdestilliert, der Rückstand mit Ligroin digeriert und abgesaugt.

Man erhält 20,5 g (47 % der Theorie) 3-(5-(2,6-Dichlor-4-trifluormethyl-phenoxy)-2-nitro-phenyl)-propensäure- methylester vom Schmelzpunkt 90°C.

Nach der im Beispiel 8 angegebenen Methode werden auch die in den folgenden Beispielen aufgeführten Stoffe der Formel (IV) hergestellt:

### Beispiel 9

Unter Verwendung von 2-Iodpropan: 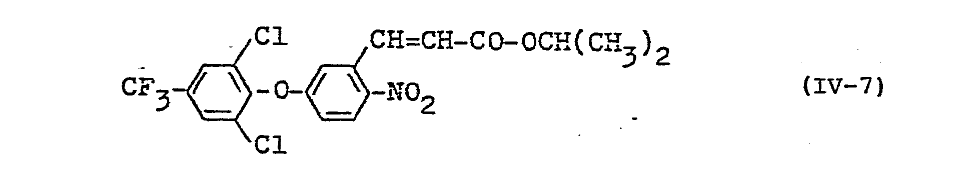 Schmelzpunkt: 102°C.

### Beispiel 10

Unter Verwendung von Ethyliodid 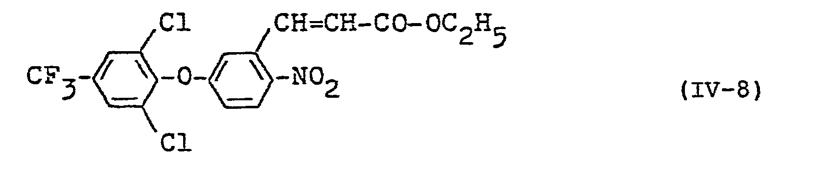 Schmelzpunkt: 86°C.

### Anwendungsbeispiele

In diesen Beispielen werden die nachstehend angegebenen Stoffe eingesetzt:

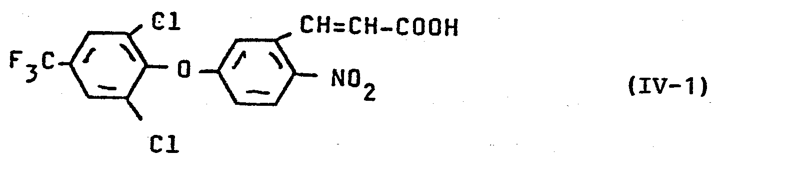

### Beispiel A

Pre-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle) 100 % = totale Vernichtung

Der Wirkstoff (IV-1) zeigt in diesem Test eine sehr gute selektive herbizide Wirksamkeit.

### Beispiel B

Post-emergence₋Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschte Wirkstoffmenge pro Flächeneinheit ausgebracht wird. Die Konzentration der Spritzbrühe wird so gewählt, daß in 2000 1 Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle) 100 % = totale Vernichtung

Der Wirkstoff (IV-1) zeigt in diesem Test eine sehr gute herbizide Wirksamkeit.

## Patentansprüche

1. 5-(2,6-Dichlor-4-trifluormethyl-phenoxy)-2-nitrobenzaldehyd-diacetacylal der Formel

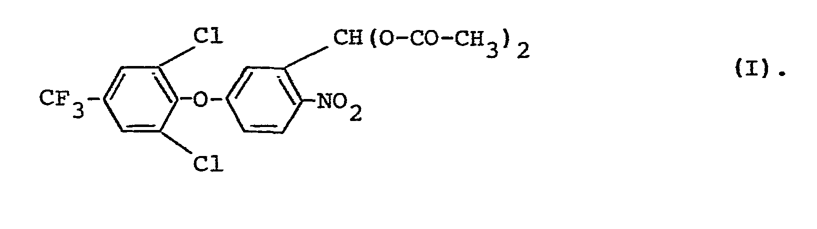

2. Verfahren zur Herstellung von 5-(2,6-Dichlor-4-trifluormethyl-phenoxy)-2-nitro-benzaldehyd-diacetacylal der Formel 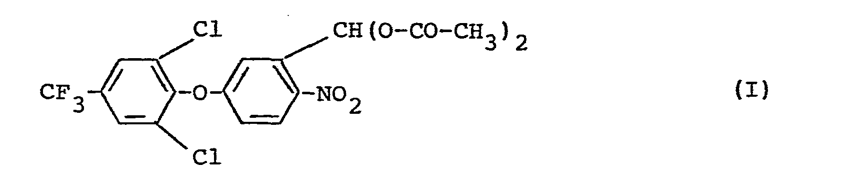 dadurch gekennzeichnet, daß man den Phenoxybenzaldehyd der Formel 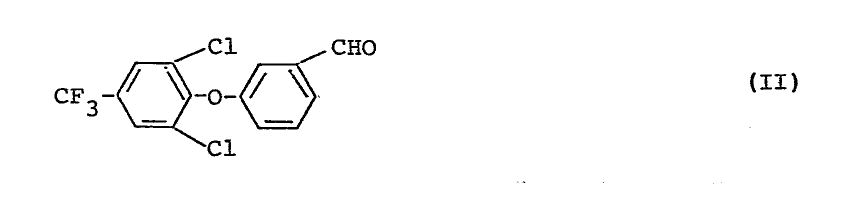 mit Acetanhydrid gegebenenfalls in Gegenwart eines Verdünnungsmittels umsetzt und anschließend mit Salpetersäure nitriert.
